# EUROPEAN PATENT SPECIFICATION

(11) **EP 3 492 046 B1**
(45) Date of publication and mention of the grant of the patent: **10.01.2024**
(21) Application number: 17306663.0
(22) Date of filing: 30.11.2017
(51) Int. Cl.: A61F 2/44, A61B 5/00, A61B 17/00, A61F 2/46, A61F 2/48

(54) **METHOD FOR MONITORING AN INTERVERTEBRAL DISC PROSTHESIS**
VERFAHREN ZUR ÜBERWACHUNG EINER BANDSCHEIBENPROTHESE
PROCÉDÉ DE SURVEILLANCE D'UNE PROTHÈSE DE DISQUE INTERVERTÉBRAL

(43) Date of publication of application: 05.06.2019
(73) Proprietor: Clariance, 62217 Beaurains (FR)
(72) Inventor: CHEVALIER, Éric, 62000 Arras (FR)
(74) Representative: Vuillermoz, Bruno

(56) References cited:
- WO-A1-96/41158
- WO-A1-2017/116346
- WO-A2-2011/149845
- WO-A2-2015/200720
- US-A1- 2005 273 170
- US-A1- 2011 213 221
- US-A1- 2013 079 680
- US-A1- 2014 275 815
- US-B2- 7 435 232

## Description

### FIELD OF INVENTION

The present invention pertains to the field of the intervertebral prosthesis. In particular, the invention relates to a method to monitor a medical device comprising an intervertebral disc prosthesis.

### BACKGROUND OF INVENTION

Artificial disc replacement is basically an implant that is used to replace a worn out or diseased intervertebral disc. The artificial disc is a metal or plastic prosthesis.

Intervertebral disc prostheses are designed to preserve movement when a spinal disc segment needs surgical attention. This surgery is normally due to a disc herniation that compresses a nerve but there are other disorders that can have indications for an ADR (bone spurs or mild degeneration). These prosthesis work by attaching to the bone of each endplate of the vertebra.

These artificial prostheses can wear out or fail like any other mechanical devices. Failure can occur from wear of the bearing surfaces or failure of the bonding between the metal and bone of the vertebra. Occasionally, the bone can be too soft to support the artificial disc and the metal surface can erode or fracture into the bone.

This generates displacement which misaligns the two bearing surfaces and causes the prosthesis surfaces to become incongruent leading to pain and reduced motion.

Providing a monitoring method which can continuously or semi-continuously monitor the physical integrity and the migration of an intervertebral prosthesis could provide welcome increases replacement clinical outcome and better patient management by an in-vivo monitoring of potential dysfunction after surgery. Document WO 2015/200720 A2 discloses devices, systems and methods for using and monitoring spinal implants.

### SUMMARY

According to a first aspect, the present invention relates to a method according to claim 1.

An advantage is that the processing steps of the signal contributes to generate a relevant indicator, for example by determining a relevant pattern and achieving correlation functions with some relevant values and for instance by comparing the different values of said pattern in a predefined duration. An advantage is to an indicator to an operator or a doctor which is representative of the physical integrity or of the offset of the intervertebral prosthesis.

In one embodiment, the intervertebral indicator comprises:
a) a stiffness of the intervertebral prosthesis, said stiffness allowing deducing a physical integrity information of said intervertebral prosthesis; and/or
b) an offset indicator of the intervertebral prosthesis.

In one embodiment, the received data measurements corresponding to mechanical vibrations of the medium.

In one embodiment, said vibration pattern is:
a) a resonant frequency; and/or
b) a spectral pattern defined in a predefined amplitude range, a predefined frequency range and a predefined phase range of a predefined vibration signal; and/or
c) a spectral density; and/or
d) at least one amplitude of the vibration signal in a predetermined frequency range beyond a predefined threshold; and/or
e) a repetition of a spectral patterns in a predefined duration.

In one embodiment, the received data corresponding to an acceleration time response.

In one embodiment, the prosthesis is an intervertebral disc prosthesis.

In one embodiment, the reference model comprises:
a) at least one predefined reference vibration patterns; and/or
b) a propagation model; and/or
c) at least a measured vibration pattern considered as a reference.

According to a second aspect, the invention relates to a medical monitoring system according to claim 7.

An advantage is to measure a criterion of the intervertebral prosthesis environment in order to give an indicator to an operator or a doctor which is representative of the physical integrity or representative of the offset of the implant. The arrangement of the sensor contributes to generate a relevant indicator.

In one embodiment, said intervertebral prosthesis does not comprise a vibration excitation transducer.

In one embodiment, the at least one vibration sensor is a piezoelectric element.

In one embodiment, the at least one vibration sensor is an accelerometer.

In one embodiment, the intervertebral prosthesis is an intervertebral disc prosthesis.

### DEFINITIONS

In the present invention, the following terms have the following meanings:
- **"Resonant frequency":** refers to the frequency at which the response amplitude is a relative maximum.
- **"Intervertebral environment of implantation":** refers to the surrounding tissues of the intervertebral prosthesis including the vertebrae surrounding the intervertebral prosthesis.
- **"Peak":** refers to a frequency or a narrow range of frequency for which the response amplitude is a relative maximum.
- **"Migration":** refers to the displacement of the prosthesis from its initial position.
- **"Vibration response":** refers to amplitude of the motion of an object or a system on its own until it returns to its resting state.
- **"Vibration pattern":** refers to a characteristic of a vibration data, said vibration pattern may be extracted by the vibration signal or by the vibration spectrum, or by another signal processing.

### DETAILED DESCRIPTION

The following detailed description will be better understood when read in conjunction with the drawings. For the purpose of illustrating, the medical device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

On a first aspect, the present invention relates to a method able to generate an intervertebral indicator giving information on the physical integrity of an intervertebral prosthesis or information on an offset of an intervertebral prosthesis.

The present method measures of the vibration response to provide information on the intervertebral prosthesis 10 being configured to be placed in an initial position between two vertebras L1, L2. and / or the intervertebral environment of implantation via an intervertebral indicator.

The method of the invention comprises the steps of:
a) receiving data from the intervertebral prosthesis 10, said data corresponding to mechanical vibration of the prosthesis or its environment, and storing said data into a computer-readable data carrier;
b) determining or calculating at least one vibration pattern of stored data;
c) comparing said vibration pattern with a at least one reference model; and
d) generating from said comparison an intervertebral indicator.

According to one embodiment, the method of the invention includes calculating a modal damping factor (MDF). Advantageously, in this invention, the frequency spectrum is used to calculate a damping factor which is proportional to the width of the resonant peak about the peak's center frequency. This embodiment can be of particular advantage, as the damping factor detected with a vibration method may provide additional information on the prosthesis displacement or on the prosthesis physical integrity.

In one embodiment, said method is a closed loop method. In one embodiment, the method is iteratively executed. In one embodiment, the present method comprises iteratively the steps a), b), c) and d).

In one embodiment, the method semi-continuously executes these steps to semi-continuously generate an intervertebral indicator. In one embodiment, the method continuously executes these steps to continuously generate an intervertebral indicator.

In one embodiment, said method further comprises the step of producing an alert signal in function of the comparing step, optionally if said comparison exceed a predetermined threshold.

According to one embodiment, said alert signal is produced by an alarm. Said alert signal can be a visual alert on the display, an audible alert, or an alert on an alert on an external device such a smartphone which is connected to the medical device.

According to a first example, said alert signal is an acoustic alarm emitted by a loudspeaker. In a second example, the alert signal is a visual alert represented on the display of the medical device. In a third example, the alert signal is a message in the form of an e-mail or a notification sent to the medical staff.

According to one embodiment the method comprises the step of determining at least one vibration pattern of stored data comprises the steps of:
- identifying a carrier frequency by applying a time-frequency transformation to the stored data;
- applying a band pass filter at a carrier frequency to create a filtered signal;
- optionally, applying a noise filter in order to filter undesired frequencies that do not represent a relevant information for evaluating the offset of the prosthesis or the physical integrity of the prosthesis;
- extracting an envelope of the filtered signal to create an extracted signal;
- applying time and frequency analysis to the extracted signal.

In one embodiment, said received data reflect mechanical vibrations of the prosthesis or its environment.

In one embodiment, said vibration pattern is a resonant frequency.

In one embodiment, the received data correspond to acceleration time response. In this embodiment, the at least one sensor place on the prosthesis include an accelerometer.

In one embodiment, the intervertebral indicator comprises a parameter reflecting the stiffness of the intervertebral prosthesis, said stiffness allowing deducing a physical integrity information of said intervertebral prosthesis and/or an offset indicator of the intervertebral prosthesis.

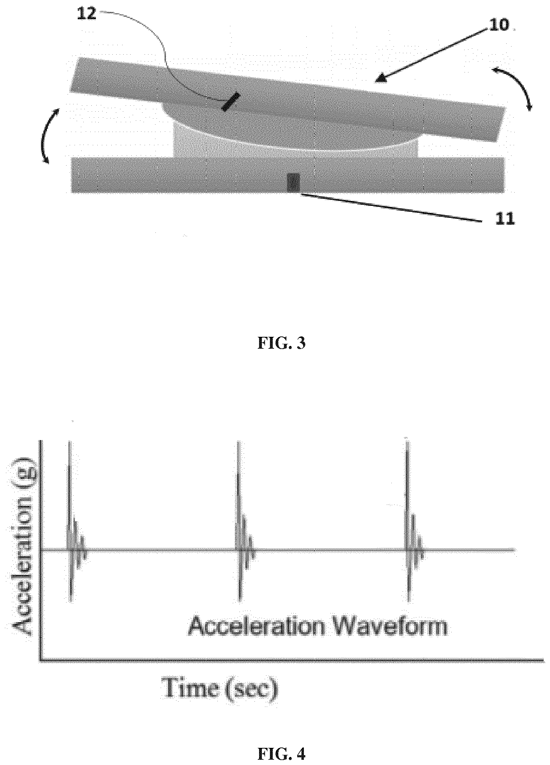

By physical integrity, it should be understood any modification in the mechanical structure of the implant 10. A modification of this mechanical structure may occur in case of, for example, a beginning of dislocation or of breakage 12, as illustrated in Fig. 3, deformation or the propagation of a crack in the implant. The monitoring of the physical integrity allows to detect any failure mode which can occur.

Furthermore, a migration of the intervertebral prosthesis, when placed between two vertebrae can be monitored by monitoring said vibration pattern. An asymmetry in the medium comprising the intervertebral prosthesis and the implantation environment leads to a variation of the vibration pattern, with comparison to an initial vibration pattern recorded upon implantation.

The method of the invention can achieve, from the vibration pattern of said intervertebral environment, to get to the user information about the migration of the implant around its initial position by the offset indicator of the intervertebral prosthesis. According to one embodiment, the present invention is able to detect a migration of the implant of at least 0.5 mm, 1 mm, 2 mm, 3 mm or at least 4 mm.

According to one embodiment, a defect or a failure in the implant, such as a crack or a fissure, can be monitored by monitoring said vibration pattern. Detecting such defect or failure permits to anticipate a break of the intervertebral prosthesis.

Because of the movement of the human body during a day, the intervertebral environment is continuously exposed to vibrations. These vibrations are associated to various frequencies. By recording continuously these frequencies with the at least one vibration sensor, the applicant found that, with enough time (from several hours to a few days), a wide range of frequencies were recorded which can allow the medical monitoring device to study the vibration response on a wide range of frequencies.

The mechanical vibrations of the medium comprising the intervertebral prosthesis and the intervertebral prosthesis environment are measured by a vibration sensor during a predetermined time. In one embodiment, the data measurements from the intervertebral prosthesis 10 are stored in a memory or in a computer-readable data carrier.

The measurement of said vibration leads to the observation of some vibration patterns.

In one embodiment, said vibration pattern is the resonant frequency. Generally, when a solid material is exposed to vibrations, the response of said material to said vibration is sensibly the same. However, there is a few specific frequencies for which the response is relevantly higher than at the other frequencies. These specific frequencies are called resonant frequencies.

According to one embodiment, combined time and frequency domain analysis of the signal generated by at least one vibration sensor accurately assess the condition of a bearing.

According to another embodiment, another vibration pattern can be used to achieve the same purpose. Said vibration pattern can be a feature of the frequency spectrum. Said vibration pattern can also be the amplitude of the signal at one predetermined frequency or the measures spectrum of frequencies.

In another embodiment, the vibration pattern is the vibration response measured at a predetermined number of frequencies. For example, the vibration pattern can be the vibration response of the intervertebral environment at sensibly 2000, 2500, 3000, 4000 and/or 4500 Hz. The vibration response at these frequencies should be compare with the vibration response of reference.

According to one embodiment, said vibration pattern is the amplitude of the signal at a predetermined frequency. According to one embodiment, said vibration pattern is a mathematical transform of the vibration response such as a Fourier transform or a wavelet transform. According to one embodiment, said vibration pattern is a variation of the frequency during the time.

In one embodiment, the method comprises the step of comparing the at least one vibration pattern with a at least one reference model.

In one embodiment, the determination of at least one vibration pattern of said received data comprises a computation of the acceleration time response.

According to one embodiment, the reference model comprises one vibration pattern of reference. In one embodiment, the reference model comprises at least two vibration patterns of reference: a first vibration pattern of reference corresponding of the vibration pattern value for a nominal functioning of prosthesis and a second vibration pattern of reference value corresponding to the vibration pattern value when a modification of the stiffness of the intervertebral prosthesis. In one embodiment, the reference model comprises a propagation model (temporal propagation).

According to one embodiment, the reference model comprises at least one vibration pattern of reference corresponding to the vibration pattern when the position of the intervertebral prosthesis in its initial position. According to one embodiment, said vibration pattern of reference can be measured and/or stored following to the implantation of the implant.

By "following to the implantation", it has to be understood here "a few minutes, a few hours or a few days after the implantation of the implant in the user body".

According to one embodiment wherein the vibration pattern is the resonant frequency. In one embodiment, the method comprises the step of scanning the vibration response measured for each vibration frequency of the measured spectrum of frequencies. In one embodiment, the method comprises the step of determining at least one resonant frequency of reference for which the vibration response is relevantly higher than for the other frequencies. According to one embodiment, the received data is a spectrum of frequency.

In one embodiment, the step of calculating the at least one resonant frequency value of said intervertebral environment, comprises the following steps of:
- from the measurements made by the vibration sensor; scanning for each frequency the vibration response of said intervertebral environment;
- identifying one or more peak of said vibration response;
- optionally recording the frequency value of the center of said at least one peak;
- optionally labelling said frequency value as the resonant frequency value.

The intervertebral environment can comprise two or at least two resonant frequencies, and these two resonant frequencies can be calculated by the medical monitoring device.

According to one embodiment, the method comprises at least two reference models, a first reference model to generate a medium indicator from a first resonant frequency and a second reference model to generate a medium indicator from a second resonant frequency.

According to one embodiment, the reference model comprises at least one equation which follows the evolution of the at least one vibration pattern in function of the medium indicator.

In one embodiment, numerical or analogical amplifiers or filters may be used in order to treat the vibration signals before extracting a vibration pattern. A correlation method, for instance using maximum likelihood criteria, may be applied with some predefined signals having some predefined patterns in order to extract some vibration patterns of the measured signal.

From the measurement of the vibration response, a computer connected to the computer-readable data carrier is able to calculate the at least one vibration pattern of the intervertebral environment and any other suitable information from the recorded data.

According to one embodiment, the reference model comprises at least one second vibration pattern of reference corresponding to the vibration pattern when the position of the intervertebral prosthesis is located at a predetermined offset of its initial position.

The reference model can also be a database.

In one embodiment, the reference model comprises some predefined vibration patterns, for example defined in a predefined amplitude range, a predefined frequency range and a predefined phase range of the signal.

In one embodiment, the reference model comprises some predefined spectral density with different values associated to different patterns.

In one embodiment, the reference model comprises a repetition of likehood patterns in a predefined duration.

According to one embodiment, said reference model is stored in the computer-readable data carrier (or a memory unit) manually by the user or by an operator. In another embodiment, said at least one reference model is measured by the at least one vibration sensor following the implantation of the implant.

According to one embodiment, the reference model comprises at least one equation which follows the evolution of the at least one vibration pattern in function of the intervertebral indicator.

According to one embodiment, the reference model comprises data from which a calculator can found the migration of the intervertebral prosthesis by the vibration pattern. The reference model can also be a database.

In one embodiment, from said comparison between the at least one vibration pattern and the at least one reference model, the method generates an intervertebral indicator.

In one embodiment, the intervertebral indicator comprises the intervertebral indicator comprises a stiffness of the intervertebral prosthesis, said stiffness allowing deducing a physical integrity information of said intervertebral prosthesis.

In one embodiment, the intervertebral indicator comprising the stiffness of the intervertebral prosthesis is a percentage between its initial stiffness and its critical stiffness. The critical stiffness may be the limit stiffness before breakage. In one embodiment, the stiffness allows to deduce a physical integrity information of said intervertebral prosthesis as the probability of breakage or an estimation of the expected lifetime of the intervertebral prosthesis.

In one embodiment, the intervertebral indicator comprises an offset indicator of the intervertebral prosthesis.

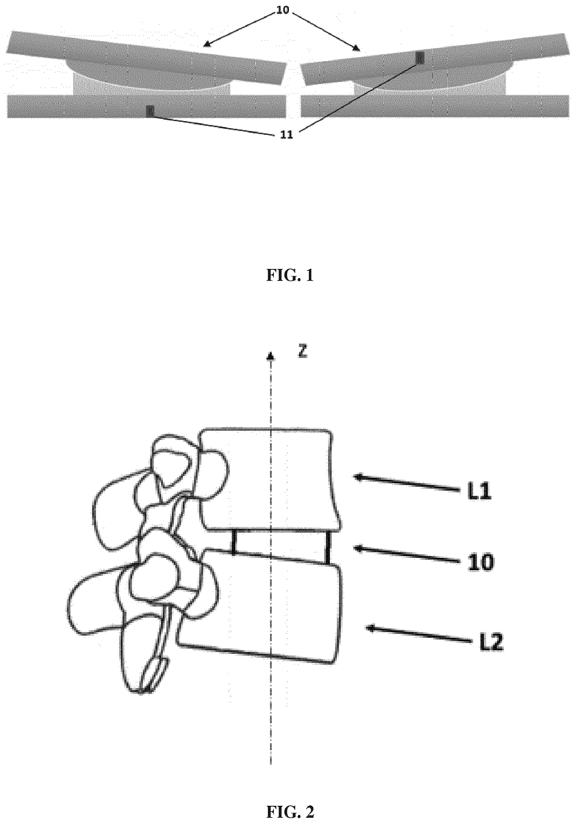

According to one embodiment, the method generates an offset indicator of the intervertebral prosthesis in function of the step of comparing the at least one vibration pattern with a reference model. In one embodiment, the offset indicator corresponding to a migration of the intervertebral prosthesis around its initial position and preferably to a migration of the intervertebral prosthesis around its initial position along the orthogonal axes to the vertebral axis z illustrated on Fig. 2.

According to one embodiment, the difference between said at least one vibration pattern and said at least one reference model is function of the migration of the intervertebral disc prosthesis around its initial position.

According to one embodiment, the difference between said at least one vibration pattern and said at least one reference model is function of evolution of the mechanical structure of the intervertebral prosthesis.

In one embodiment, the method is able to generate two intervertebral indicators.

According to one embodiment, the at least one reference model comprises a first reference model to generate a first intervertebral indicator comprises a stiffness of the intervertebral prosthesis.

According to one embodiment, the at least one reference model comprises a second reference model to generate a second offset indicator of the intervertebral prosthesis around its initial position.

According to one embodiment, among the two following indicators: the first intervertebral indicator and the second offset indicator, one of said indicator is known and is used to calculate the other one.

In one embodiment, after generating a first intervertebral indicator comprises the offset indicator of the interval prosthesis is known, a variation of the vibration pattern should be caused by the evolution of the stiffness of the intervertebral prosthesis.

According to one embodiment, the step of comparing the at least one vibration pattern and the reference model comprising the steps of calculating the difference between the at least one vibration pattern value and the reference model value and comparing said difference to a predetermined threshold. According to one embodiment, said predetermined threshold is ranging from 1% to 7% of the reference model value, preferably ranging from 0.5% to 10% or ranging from 0.5% to 20%.

According to one embodiment, the vibration pattern is determined by applying to the measurement data a Discrete Wavelet Transform (DWT) to predict the occurrence of a spalling or a wear. The DWT yields information on both the time and frequency characteristics of the input signal, and is particularly helpful in detecting subtle time localized changes. By applying the DWT to the vibration signals and comparing the DWT coefficient at various resolutions, the transient effect of spalling or wear can be effectively and sensitively identified. Prediction of spalling or wear is possible through inspecting the trends of the coefficient values.

According to one embodiment, the reference model is a DWT of reference. In one embodiment, the vibration pattern is a functional mode and the reference model is a functional mode of reference.

In one embodiment, the extracted signal is then compared to an extracted signal of reference to generate intervertebral indicator.

In one embodiment, at least one vibration pattern is determined from said extracted signal.

In one embodiment, the vibration pattern is a function of the damping factor and said reference model is a function of damping factor of reference.

In one embodiment, the intervertebral prosthesis described in this method is an intervertebral disc prosthesis.

On a second aspect, the present invention relates to a medical monitoring system comprising an intervertebral prosthesis able to execute the method according to the first aspect of the present invention.

In one embodiment, the present invention involves the direct structural integration of at least one sensing element such as a vibration sensor, and telemetric data communication means into the said disc prosthesis environment to provide self-monitoring capabilities. Once implanted, the sensing element continuously or on demand measures the real-time fluctuations of the intervertebral disc prosthesis load variations and vibrations. If predetermined threshold values representing critical disc prosthesis operational parameters are exceeded an alarm can be triggered.

The intervertebral prosthesis 10 comprises at least one vibration sensor 11 configured to measure the mechanical vibrations of a medium comprising said intervertebral disc prosthesis 10 and an intervertebral prosthesis environment. The medical monitoring system further comprises means for transmitting said measurements from the intervertebral prosthesis to an external device.

In one embodiment, the medical monitoring system comprises:
a) an intervertebral prosthesis 10 comprises at least one vibration sensor 11 configured to measure the mechanical vibrations of a medium comprising said intervertebral disc prosthesis 10 and an intervertebral prosthesis environment;
b) a computer-readable data carrier configured to receive and store the data measurement from the at least one vibration sensor;
c) a medical monitoring device connected to the at least one vibration sensor and comprising at least one calculator.

In one embodiment, said medical monitoring system is able to execute the method according to the first aspect of the present invention.

In one embodiment, the medical monitoring system comprises:
a) an intervertebral prosthesis comprising a vibration sensor 11 and a transmitter for transmitting data;
b) a receiver for receiving data from the intervertebral prosthesis, corresponding to mechanical vibrations of a medium; and
c) a calculator computing from the received data an intervertebral indicator by:
   i. determining at least one vibration pattern of said received data;
   ii. comparing said at least one vibration pattern with at least one reference model;
   iii. generating an intervertebral indicator (MI) in function of the comparing step.

In one embodiment, the medical monitoring system comprises:
a) an intervertebral prosthesis comprising a vibration sensor 11 and a transmitter for transmitting data;
b) a receiver for receiving data from the intervertebral prosthesis, corresponding to mechanical vibrations of a medium; and
c) a calculator computing from the received data an intervertebral indicator by:
   i. receiving data measurements from the intervertebral prosthesis and storing said data into a computer-readable data carrier;
   ii. determining at least one vibration pattern of said stored data;
   iii. comparing said at least one vibration pattern with at least one reference model;
   iv. generating from said comparison an intervertebral indicator.

In one embodiment, the intervertebral prosthesis is an intervertebral disc prosthesis. In one embodiment, the intervertebral disc prosthesis is made of metal, cobalt-chromium alloy (CoCr), titanium (Ti), diamond-like carbon (DLC) coatings, silicone or an assembly of polymers. According to one embodiment, the intervertebral disc prosthesis is made by 3D-printing. In the following description, the word implant can also be used to refer to the intervertebral disc prosthesis.

As illustrated on Fig. 2, the intervertebral disc prosthesis 10 is configured to be inserted between the two vertebrae (L1 and L2) allowing a mobile preservation of the spine level.

According to one embodiment illustrated on Fig. 1, the intervertebral disc prosthesis 10 comprises (ball and socket design type) two plates and inlays. An alternative well-known design is a one-piece bearing design type consisting in one cushion between two plates to more adequately mimic the natural disc behavior through the implant.

In one embodiment, the intervertebral disc prosthesis 10 is configured to be fixed to the superior and the inferior vertebrae between which it is configured to be placed.

The intervertebral disc prosthesis 10 further comprises at least one vibration sensor 11. The at least one vibration sensor 11 is configured to measure a vibration response of a medium comprising said intervertebral disc prosthesis and said intervertebral environment of implantation. According to one embodiment, the at least one vibration sensor 11 is arranged to measure a vibration frequency value and a vibration response value (i.e. amplitude) of said medium.

According to one embodiment, at least one plate comprises at least one vibration sensor 11.

According to one embodiment, the at least one vibration sensor 11 is placed on the horizontal side of the intervertebral disc prosthesis. By horizontal side of the intervertebral disc prosthesis, it should be understood the surface which is directed to the adjacent vertebral endplate.

According to one preferred embodiment, the at least one vibration sensor 11 is a piezoelectric element. The piezoelectric vibration sensor, when exposed to a mechanical stress or a deformation, can provide an electric field. The current provided by the piezoelectric element is function of the intensity of the deformation (or stress). A piezoelectric element is so able to measure the amplitude and the frequency of the vibrations.

In one embodiment, the frame of the intervertebral prosthesis is made of metal, graphite, or bone, preferably made of titanium or polyether ether ketone (PEEK). According to one embodiment, the frame of the intervertebral prosthesis is made of porous titanium. According to one embodiment, the frame of the intervertebral prosthesis is made by 3D-printing. Preferably, the intervertebral prosthesis is cylinder in shape or rectangular in shape. In the following description, the word implant can also be used to refer to the intervertebral prosthesis.

In general, vertebrae are exposed to a multitude of vibration during patient movements. Statistically, during a sufficiently long period of time, the medium comprising the intervertebral prosthesis and intervertebral environment of implantation will be exposed to a large spectrum of frequencies.

In one embodiment, the at least one vibration sensor 11 is configured to measure a vibration response value of the medium. Indeed, the vibration sensor 11 is able to create an electric charge in response to applied mechanical stress. When a vibration occurs, said vibration sensor 11 measures the vibration response of said system.

According to one embodiment, the at least one vibration sensor 11 is configured to measure a vibration response of the medium at a frequency ranging of from 20 Hz to 10000 Hz, preferably from 30 Hz to 7000 Hz, more preferably from 40 Hz to 5000 Hz.

According to one embodiment, the at least one vibration sensor is configured to measure a vibration frequency of the intervertebral prosthesis at a frequency ranging of from 20 Hz to 10000 Hz, preferably from 30 Hz to 7000 Hz, more preferably from 40 Hz to 5000 Hz.

In one embodiment, the term "measure" as to be understood by transforming the mechanical stress in electric current. In one embodiment, the measurement is provided by an impedance meter which can be in the implant 11 or in an external device. In one embodiment, the at least one vibration sensor 11 comprises at least one piezoelectric element and means to measure current, voltage and/or impedance provided by the at least one piezoelectric element. The at least one piezoelectric element and said means to measure current, voltage and/or impedance provided by the at least one piezoelectric element are connected each other by wires or wireless.

According to one preferred embodiment, the at least one vibration sensor 11 is an accelerometer element. The said accelerometer is an electromechanical device that will measure both static (gravity) and dynamic (motion or vibration) accelerations.

When the patient moves, specific constraints apply on vertebrae: extension, flexion, compression, torsion, etc. When a structural change or a displacement occurs in the intervertebral prosthesis, constraint such as extension leads to different vibration patterns. The integration of an accelerometer into the said intervertebral prosthesis 10 provides useful vibration patterns by the measurement of the acceleration time response: with a 3-axis accelerometer, the signal from the horizontal, vertical and axial direction can be captured; with an only one or 2 axis designs, the said accelerometer orientation is then critical.

According to one embodiment, said accelerometer can provide patient position and movement information for postoperative process.

According to one embodiment, the signal generated by the at least one vibration sensor 11 allows obtaining a frequency spectrum. In one embodiment, the signal generated by the at least one vibration sensor 11 is transformed by a Fourier transform or a wavelet transform to obtain a frequency spectrum.

According to one embodiment, this invention includes means for calculating a modal damping factor (MDF). Advantageously, in this invention, the frequency spectrum is used to calculate a damping factor which is proportional to the width of the resonant peak about the peak's center frequency.

According to one embodiment, said intervertebral prosthesis does not comprise a vibration excitation transducer. Indeed, the waves are naturally generated movement of the spinal column.

The medical monitoring system comprises a computer-readable data carrier. According to one embodiment, the intervertebral prosthesis comprises an external device connected to said vibration sensor 11 and comprises a calculator able to execute the method according to the first aspect of the present invention. In one embodiment, the medical monitoring system further comprises a computer-readable data carrier for storing the data measurements received from the vibration sensor. In on embodiment, said computer-readable data carrier and said calculator are required to analyze the measurement obtained by the vibration sensor 11 and to generate an intervertebral indicator. In one embodiment, said indicator allows deducing a feedback on the bearing conditions of said intervertebral disc prosthesis and / or on the migration of the implant. According to a preferred embodiment, the medical monitoring system comprises an external device and said external device comprises said computer-readable data carrier and said calculator. In one embodiment, the computer-readable data carrier is located in the intervertebral prosthesis and stored measurement data may be transmit by wireless. In one embodiment, the medical monitoring system further comprises a wireless interface for transmitting said stored data to an external device. According to one embodiment, said external device is configured to be placed outside the body of the user. According to one embodiment, said external device is a belt, preferably an abdominal belt.

According to one embodiment, said external device further comprises an impedance meter, and/ or a user interface such as a display. According to one embodiment, said interface is required to allow the user to provide its measurement parameters and to display the at least one generated intervertebral indicator.

The medical monitoring system according to the present invention further comprises means for transmitting said measurements from the intervertebral prosthesis to the computer-readable data carrier. In one embodiment, said means comprise at least one transmitter and at least one receiver. In one embodiment, the implant and the external device both comprises means for transmitting and receiving said measurements. In one embodiment, the medical monitoring system comprises means for transmitting said measurements and said means allow a bi-directional communication between the user interface and the implant, or between the computer-readable data carrier and the implant, or between the computer-readable data carrier and the vibration sensor.

According to one embodiment, both the external device and the intervertebral prosthesis comprises means for data transmission and data reception connected one to the other.

According to one embodiment, said external device comprises a receiver and is configured to be placed on the back of the user body, at the level of the implanted intervertebral prosthesis to improve transmissions.

According to one embodiment, the external device comprises means for providing energy. According to one embodiment, the external device is able to provide the energy to supply the intervertebral prosthesis. According to one embodiment, the external device comprises a wireless transmitter connected to a power source.

According to one embodiment, the intervertebral prosthesis comprises at least one receiver. According to another embodiment, the medical monitoring system comprises a receiver connected with wire to the intervertebral prosthesis. According to one embodiment, the wireless transmitter connected to a power source conveys the field energy across an intervening space to said receiver, and said receiver converts back said field energy to an electrical current.

In said embodiment, the medical monitoring system can provide energy to the means for transmitting data.

In one embodiment, the intervertebral prosthesis comprises at least a receiver for receiving energy and at least one transmitter to send the measurement data to the external device.

According to one embodiment non-illustrated, the intervertebral prosthesis is connected to said receiver for receiving energy and connected to said at least one transmitter to send the measurement data to the external device by a wire.

According to one embodiment, the intervertebral prosthesis does not comprise an implanted memory. In one embodiment, the intervertebral prosthesis does not comprise an implanted battery.

According to one embodiment, the medical device further comprises a memory unit. Preferably, said memory unit is comprised in the external device.

In one embodiment, said memory unit comprises data for calibration, and permits the calculator to calculate a variation of vibration pattern of the disc prosthesis and / or a migration of the intervertebral prosthesis from the at least one resonant frequency.

According to one embodiment, said medical monitoring system further comprises a display. Said display being configured to display the intervertebral indicator or a calculated variation of the vibration pattern or of a functional mode of the prosthesis.

According to one embodiment, the medical monitoring system further comprises an alarm. Said alarm can be a visual or an audible alarm.

In another aspect, the invention relates to a medical device comprising an intervertebral prosthesis 10 and a computer-readable data carrier, said intervertebral prosthesis 10 being configured to be placed in an initial position between two vertebras L1, L2. Said intervertebral prosthesis 10 comprises at least one vibration sensor 11 configured to measure a vibration response of a medium comprising said intervertebral prosthesis 10 and an intervertebral environment of implantation. Said medical device further comprises means for transmitting said measurements from the intervertebral prosthesis 10 to the computer-readable data carrier. Said computer-readable data carrier comprises at least one vibration pattern of reference and instructions which, when executed by a computer, cause the computer to carry out the step of:
a) determining at least one vibration pattern of said system from the measured vibration response;
b) comparing said at least one vibration pattern and said vibration pattern of reference;
c) determining from said comparison, physical integrity information of the intervertebral prosthesis 10 and / or a migration of the intervertebral prosthesis 10.

While various embodiments have been described and illustrated, the detailed description is not to be construed as being limited hereto. Various modifications can be made to the embodiments by those skilled in the art without departing from the scope of the disclosure as defined by the claims.

### BRIEF DESCRIPTION OF THE DRAWINGS

**Figure 1** is a drawing of the intervertebral disc prosthesis according to one embodiment of the present invention.
**Figure 2** is a drawing of the intervertebral disc prosthesis according to one embodiment of the present invention between two vertebrae.
**Figure 3** is a drawing of the intervertebral disc prosthesis comprising a failure in the disc frame.
**Figure 4** is a graph of a generated specific high frequency damped oscillation captured by the accelerometer when the intervertebral disc prosthesis comprises a failure.

### REFERENCES

10 - Intervertebral disc prosthesis
11 - Vibration sensor
L1 - Vertebrae
L2 - Vertebrae

## Claims

1. A method for generating a dynamic intervertebral indicator of an intervertebral prosthesis (10), said intervertebral prosthesis (10) being configured to be placed in an initial position between two vertebras (L1, L2), said intervertebral prosthesis (10) comprising at least one vibration sensor (11) configured to measure the mechanical vibrations of a medium comprising said intervertebral prosthesis (10) and an intervertebral prosthesis environment, wherein the method comprises the steps of:
a) receiving data measurements from the intervertebral prosthesis (10) and storing said data into a computer-readable data carrier;
b) determining at least one vibration pattern of stored data;
c) comparing the at least one vibration pattern with a at least one reference model; and
d) generating from said comparison an intervertebral indicator;
and **characterized in that** the said vibration pattern is:
e) a resonant frequency; and/or
f) a spectral pattern defined in a predefined amplitude range, a predefined frequency range and a predefined phase range of a predefined vibration signal; and/or
g) a spectral density; and/or
h) at least one amplitude of the vibration signal in a predetermined frequency range beyond a predefined threshold; and/or
i) a repetition of a spectral patterns in a predefined duration.

2. A method according to claim 1, wherein the intervertebral indicator comprises:
a) a stiffness of the intervertebral prosthesis, said stiffness allowing deducing a physical integrity information of said intervertebral prosthesis; and/or
b) an offset indicator of the intervertebral prosthesis.

3. A method according to claim 1 or claim 2, wherein the received data measurements corresponding to mechanical vibrations of the medium.

4. A method according to anyone of claims 1 to 3, wherein the received data measurements corresponding to an acceleration time response.

5. A method according to anyone of claims 1 to 4, wherein the prosthesis is an intervertebral disc prosthesis.

6. A method according to anyone of claims 1 to 5, wherein the reference model comprises:
a) at least one predefined reference vibration patterns; and/or
b) a propagation model; and/or
c) at least a measured vibration pattern considered as a reference.

7. A medical monitoring system comprising:
a) an intervertebral prosthesis (10) comprises at least one vibration sensor (11) configured to measure the mechanical vibrations of a medium comprising said intervertebral disc prosthesis (10) and an intervertebral prosthesis environment;
b) a computer-readable data carrier configured to receive and store the data measurement from the at least one vibration sensor; and
c) a medical monitoring device connected to the at least one vibration sensor and comprising at least one calculator;
said medical monitoring system being able to execute the method according to anyone of claims 1 to 6.

8. A medical monitoring system according to claim 7, wherein said intervertebral prosthesis (10) does not comprise a vibration excitation transducer.

9. A medical monitoring system according to claim 7 or claim 8, wherein the at least one vibration sensor (11) is a piezoelectric element.

10. A medical monitoring system according to claim 7 or claim 8, wherein the at least one vibration sensor (11) is an accelerometer.

11. A medical monitoring system according to anyone of claims 7 to 10, wherein the intervertebral prosthesis is an intervertebral disc prosthesis.

## Patentansprüche

1. Verfahren zum Erzeugen eines dynamischen Zwischenwirbelkennwerts einer Zwischenwirbelprothese (10), wobei die Zwischenwirbelprothese (10) dafür ausgelegt ist, in einer Anfangsposition zwischen zwei Wirbeln (L1, L2) angeordnet zu werden, wobei die Zwischenwirbelprothese (10) mindestens einen Schwingungssensor (11) umfasst, der dafür ausgelegt ist, die mechanischen Schwingungen eines Mediums zu messen, welches die Zwischenwirbelprothese (10) und eine Zwischenwirbelprothesenumgebung umfasst, wobei das Verfahren die folgenden Schritte umfasst:
a) Empfangen von Datenmesswerten von der Zwischenwirbelprothese (10) und Speichern dieser Daten auf einem maschinenlesbaren Datenträger;
b) Bestimmen mindestens eines Schwingungsmusters von gespeicherten Daten;
c) Vergleichen des mindestens einen Schwingungsmusters mit mindestens einem Bezugsmodell; und
d) Erzeugen, ausgehend von dem Vergleich, eines Zwischenwirbelkennwerts;
und **dadurch gekennzeichnet ist, dass** es sich bei dem Schwingungsmuster um Folgendes handelt:
e) eine Resonanzfrequenz; und/oder
f) ein spektrales Muster, das in einem vorbestimmten Amplitudenbereich, einem vorbestimmten Frequenzbereich und einem vorbestimmten Phasenbereich eines vorbestimmten Schwingungssignals festgelegt wird; und/oder
g) eine spektrale Dichte; und/oder
h) mindestens eine Amplitude des Schwingungssignals in einem vorbestimmten Frequenzbereich jenseits eines vorbestimmten Schwellenwerts; und/oder
i) eine Wiederholung eines spektralen Musters innerhalb einer vorbestimmten Dauer.

2. Verfahren gemäß Anspruch 1, wobei der Zwischenwirbelkennwert Folgendes umfasst:
a) eine Steifigkeit der Zwischenwirbelprothese, wobei aus der Steifigkeit eine Information zur physischen Unversehrtheit der Zwischenwirbelprothese abgeleitet werden kann; und/oder
b) einen Kennwert zum Versatz der Zwischenwirbelprothese.

3. Verfahren gemäß Anspruch 1 oder Anspruch 2, wobei die empfangenen Datenmesswerte mechanischen Schwingungen des Mediums entsprechen.

4. Verfahren gemäß einem beliebigen der Ansprüche 1 bis 3, wobei die empfangenen Datenmesswerte einer Beschleunigungszeitreaktion entsprechen.

5. Verfahren gemäß einem beliebigen der Ansprüche 1 bis 4, wobei es sich bei der Prothese um eine Bandscheibenprothese handelt.

6. Verfahren gemäß einem beliebigen der Ansprüche 1 bis 5, wobei das Bezugsmodell Folgendes umfasst:
a) mindestens ein vorbestimmtes Bezugsschwingungsmuster; und/oder
b) ein Ausbreitungsmodell; und/oder
c) mindestens ein gemessenes Schwingungsmuster, auf welches Bezug genommen wird.

7. Medizinisches Überwachungssystem, umfassend:
a) eine Zwischenwirbelprothese (10), die mindestens einen Schwingungssensor (11) umfasst, welcher dafür ausgelegt ist, die mechanischen Schwingungen eines Mediums zu messen, welches die Bandscheibenprothese (10) und eine Zwischenwirbelprothesenumgebung umfasst;
b) einen maschinenlesbaren Datenträger, der dafür ausgelegt ist, die Datenmesswerte von dem mindestens einen Schwingungssensor zu empfangen und zu speichern; und
c) eine medizinische Überwachungsvorrichtung, die an den mindestens einen Schwingungssensor angeschlossen ist und mindestens ein Steuergerät umfasst;
wobei das medizinische Überwachungssystem dazu befähigt ist, das Verfahren gemäß einem beliebigen der Ansprüche 1 bis 6 auszuführen.

8. Medizinisches Überwachungssystem gemäß Anspruch 7, wobei die Zwischenwirbelprothese (10) keinerlei Wandler zur Schwingungsanregung umfasst.

9. Medizinisches Überwachungssystem gemäß Anspruch 7 oder Anspruch 8, wobei es sich bei dem mindestens einen Schwingungssensor (11) um ein piezoelektrisches Element handelt.

10. Medizinisches Überwachungssystem gemäß Anspruch 7 oder Anspruch 8, wobei es sich bei dem mindestens einen Schwingungssensor (11) um einen Beschleunigungsmesser handelt.

11. Medizinisches Überwachungssystem gemäß einem beliebigen der Ansprüche 7 bis 10, wobei es sich bei der Zwischenwirbelprothese um eine Bandscheibenprothese handelt.

## Revendications

1. Procédé pour générer un indicateur intervertébral dynamique d'une prothèse intervertébrale (10), ladite prothèse intervertébrale (10) étant configurée pour être placée dans une position initiale entre deux vertèbres (L1, L2), ladite prothèse intervertébrale (10) comprenant au moins un capteur de vibrations (11) configuré pour mesurer les vibrations mécaniques d'un milieu comprenant ladite prothèse intervertébrale (10) et un environnement de prothèse intervertébrale, dans lequel le procédé comprend les étapes consistant à :
a) recevoir des mesures de données provenant de la prothèse intervertébrale (10) et stocker lesdites données dans un support de données lisible par ordinateur ;
b) déterminer au moins un motif vibratoire de données stockées ;
c) comparer le au moins un motif vibratoire avec un au moins un modèle de référence ; et
d) générer à partir de ladite comparaison un indicateur intervertébral ;
et **caractérisé en ce que** ledit motif vibratoire est :
e) une fréquence de résonance ; et/ou
f) un motif spectral défini dans une plage d'amplitude prédéfinie, une plage de fréquence prédéfinie et une plage de phase prédéfinie d'un signal vibratoire prédéfini ; et/ou
g) une densité spectrale ; et/ou
h) au moins une amplitude du signal vibratoire dans une plage de fréquence prédéterminée au-delà d'un seuil prédéfini ; et/ou
i) une répétition d'un motif spectral sur une durée prédéfinie.

2. Procédé selon la revendication 1, dans lequel l'indicateur intervertébral comprend :
a) une rigidité de la prothèse intervertébrale, ladite rigidité permettant de déduire des informations d'intégrité physique de ladite prothèse intervertébrale ; et/ou
b) un indicateur de décalage de la prothèse intervertébrale.

3. Procédé selon la revendication 1 ou la revendication 2, dans lequel les mesures de données reçues correspondent à des vibrations mécaniques du milieu.

4. Procédé selon l'une quelconque des revendications 1 à 3, dans lequel les mesures de données reçues correspondent à une réponse de temps d'accélération.

5. Procédé selon l'une quelconque des revendications 1 à 4, dans lequel la prothèse est une prothèse de disque intervertébral.

6. Procédé selon l'une quelconque des revendications 1 à 5, dans lequel le modèle de référence comprend :
a) au moins un motif vibratoire de référence prédéfini ; et/ou
b) un modèle de propagation ; et/ou
c) au moins un motif vibratoire mesuré considéré comme une référence.

7. Système de surveillance médical comprenant :
a) une prothèse intervertébrale (10) comprenant au moins un capteur de vibrations (11) configuré pour mesurer les vibrations mécaniques d'un milieu comprenant ladite prothèse de disque intervertébral (10) et un environnement de prothèse intervertébrale ;
b) un support de données lisible par ordinateur configuré pour recevoir et stocker la mesure de données provenant du au moins un capteur de vibrations ; et
c) un dispositif de surveillance médical connecté au au moins un capteur de vibrations et comprenant au moins un calculateur ;
ledit système de surveillance médical étant capable d'exécuter le procédé selon l'une quelconque des revendications 1 à 6.

8. Système de surveillance médical selon la revendication 7, dans lequel ladite prothèse intervertébrale (10) ne comprend pas de transducteur d'excitation vibratoire.

9. Système de surveillance médical selon la revendication 7 ou la revendication 8, dans lequel le au moins un capteur de vibrations (11) est un élément piézoélectrique.

10. Système de surveillance médical selon la revendication 7 ou la revendication 8, dans lequel le au moins un capteur de vibrations (11) est un accéléromètre.

11. Système de surveillance médical selon l'une quelconque des revendications 7 à 10, dans lequel la prothèse intervertébrale est une prothèse de disque intervertébral.
